# FASCICULE DE BREVET EUROPEEN

(11) **EP 3 107 980 B1**
(45) Date de publication et mention de la délivrance du brevet: **01.01.2020**
(21) Numéro de dépôt: 15704004.9
(22) Date de dépôt: 12.02.2015
(51) Int. Cl.: C10G 50/00, C07C 2/12, B01J 38/16, B01J 29/18, B01J 29/40, B01J 29/65, B01J 29/70, B01J 29/80, B01J 29/90

(54) **PROCEDE D'OLIGOMERISATION D'OLEFINES UTILISANT UN CATALYSEUR COMPRENANT UNE ZEOLITHE ET UN LIANT ALUMINE AYANT SUBI UNE ETAPE DE TRAITEMENT THERMIQUE SPECIFIQUE**
VERFAHREN ZUR OLIGOMERISIERUNG VON OLEFINEN UNTER VERWENDUNG EINES KATALYSATORS MIT EINEM ZEOLITH UND ALUMINIUMBASIERTEM BINDEMITTEL MIT SPEZIFISCHEM WÄRMEBEHANDLUNGSSCHRITT
METHOD FOR OLIGOMERIZING OLEFINS USING A CATALYST THAT COMPRISES A ZEOLITE AND AN ALUMINA-BASED BINDER AND HAS UNDERGONE A SPECIFIC THERMAL TREATMENT STEP

(30) Priorité: 19.02.2014 FR 1451310
(43) Date de publication de la demande: 28.12.2016
(73) Titulaire: IFP Energies nouvelles, 92500 Rueil-Malmaison (FR)
(72) Inventeur: CADRAN, Nicolas, 69600 Oullins (FR); BAZER-BACHI, Delphine, 69540 Irigny (FR); HUGUES, Francois, 69390 Vernaison (FR)
(74) Mandataire: IFP Energies nouvelles
(86) Numéro de dépôt international: PCT/EP2015/052956
(87) Numéro de publication internationale: WO 2015/124490

(56) Documents cités:
- EP-A2- 0 034 444
- WO-A1-2009/105248
- US-A- 4 642 404
- US-A- 5 284 989
- D L ; Trimm ET AL: "Chemistry of Olefin Oligomerization over ZSM-5 Catalyst", Ind. Eng. Chem. Res. Tech. Biotechnol, 1 janvier 1988 (1988-01-01), pages 565-570, XP055157416, Extrait de l'Internet: URL:http://pubs.acs.org/doi/pdf/10.1021/ie 00076a006 [extrait le 2014-12-08]
- ZHANG ET AL: "The performance of HMCM-22 zeolite catalyst on the olefin alkylation thiophenic sulfur in gasoline", CATALYSIS COMMUNICATIONS, ELSEVIER SCIENCE, AMSTERDAM, NL, vol. 9, no. 1, 9 novembre 2007 (2007-11-09), pages 60-64, XP022338938, ISSN: 1566-7367, DOI: 10.1016/J.CATCOM.2007.05.026

## Description

### Domaine de l'invention

L'invention concerne l'oligomérisation d'une charge oléfinique pour la production de distillats moyens, et en particulier, l'utilisation d'un catalyseur comprenant au moins une zéolithe possédant des ouvertures de pores contenant 10 ou 12 atomes d'oxygènes (10MR ou 12MR) mise en forme avec un liant aluminique dans un procédé d'oligomérisation des oléfines, ledit catalyseur ayant subi une étape de traitement spécifique.

### Étude de l'art antérieur

Les procédés d'oligomérisation des oléfines légères destinés à produire des oléfines de plus haut poids moléculaire sont largement utilisés en raffinage et pétrochimie dans le but de valoriser les oléfines légères en bases pour carburants de type essence, kérosène ou gazole, ou bien en solvants. Ces réactions d'oligomérisation sont conduites en présence d'un catalyseur, le plus souvent un catalyseur solide. Les oléfines se combinent en dimères, trimères, tétramères, etc., le degré d'oligomérisation dépendant du type de catalyseur utilisé et des conditions opératoires de température, de pression et de débit de charge. L'avantage du procédé d'oligomérisation, par rapport à d'autres procédés bien connus dans le domaine du raffinage et de la pétrochimie conduisant à la même gamme de produits, réside dans le fait que les produits obtenus ne contiennent pas de soufre et contiennent très peu de composés aromatiques. Les catalyseurs d'oligomérisation solides souvent cités dans la littérature sont des catalyseurs de type acide phosphorique solide (US 2.913.506 et US 3.661.801), silice-alumine (US 4.197.185, US 4.544.791 et EP 0.463.673), zéolithe (US 4.642.404 et US 5.284.989) ou bien, dans une moindre mesure, de type hétéropolyanion (IN 170903).

Les catalyseurs zéolithiques sont les plus utilisés, et en particulier à base de zéolithe ZSM-5.

La demande de brevet US2009/0149684 décrit un procédé comprenant la mise en contact d'oléfines avec un lit de garde pour produire un flux d'oléfines prétraité et la mise en contact dudit flux prétaité avec un catalyseur comprenant une zéolithe et en particulier une mordénite dans une zone d'oligomérisation. Le document est muet sur l'utilisation d'un liant dans la mise en forme du catalyseur et sur un éventuel traitement dudit catalyseur.

Le brevet US 7 271 304 décrit un procédé de production de coupe hydrocarbonée bouillant à une température compatible avec la coupe gazole comprenant la mise en contact d'une charge oléfinique comprenant 3 à 8 atomes de carbones avec un catalyseur d'oligomérisation acide zéolithique, en particulier à base de ZSM-5. Le document est muet sur l'utilisation d'un liant dans la mise en forme du catalyseur à base de ZSM-5 et sur un éventuel traitement dudit catalyseur.

Quann et al: "Chemistry of Olefin Oligomerization over ZSM-5 Catalyst" décrit un procédé d'oligomérisation d'oléfines légères C₃-C₆.

Une des difficultés rencontrées est la faible productivité des catalyseurs utilisés dans l'art antérieur et leur faible stabilité temporelle. La durée de cycle desdits catalyseurs étant limitée à quelques jours, une régénération fréquente est nécessaire pour maintenir l'activité. Il est donc important que le système conserve l'essentiel de ses performances au cours de ces cycles qui constituent sa durée de vie.

Diverses méthodes ont été employées pour augmenter la stabilité du catalyseur mis en œuvre dans les procédés d'oligomérisation de coupes oléfiniques. Le brevet US 5 672 800 décrit en particulier le traitement d'une charge oléfinique contenant une quantité d'eau comprise entre 0,05 et 0,25 % molaire par rapport à la quantité d'hydrocarbure dans la charge. La conversion des oléfines et la durée de vie du catalyseur utilisé sont améliorées par rapport à un procédé n'utilisant pas une charge hydratée présentant une telle quantité d'eau.

En cherchant à améliorer les performances de la réaction d'oligomérisation, la demanderesse a mis au point un procédé d'oligomérisation pour la production de distillats moyens, mettant en œuvre un catalyseur comprenant au moins une zéolithe possédant des ouvertures de pores contenant 10 ou 12 atomes d'oxygènes (10MR ou 12MR) mise en forme avec un liant aluminique, ledit catalyseur ayant subi au moins une étape de traitement thermique opérée sous un flux d'air humide contenant entre 1 et 20% poids d'eau, sous un débit d'air humide compris entre 0,1 et 10 NL/h/g_{cat}, à une température comprise entre 450 et 700°C et pendant une durée comprise entre 1 et 10h, ladite étape de traitement thermique étant opérée après l'étape de mise en forme de la zéolithe avec le liant aluminique.

De manière surprenante, l'utilisation dans un procédé d'oligomérisation des oléfines, d'un tel catalyseur ayant subi une étape de traitement thermique opérant dans des conditions opératoires spécifiques, permet d'augmenter le nombre de cycles pendant la durée de vie du catalyseur, et/ou permet l'obtention d'une productivité accrue pendant la durée de vie du catalyseur.

### Résumé de l'invention

La présente invention décrit un procédé d'oligomérisation d'une charge oléfinique selon la revendication 1.

Un avantage de la présente invention utilisant un catalyseur ayant subi un tel traitement thermique, est notamment de permettre de gagner en nombre de cycles pendant la durée de vie du catalyseur, et/ou de permettre une productivité accrue pendant la durée de vie du catalyseur.

### Description détaillée de l'invention

Selon l'invention, le catalyseur utilisé dans le procédé selon la présente invention comprend au moins une zéolithe possédant des ouvertures de pores contenant 10 ou 12 atomes d'oxygènes (10MR ou 12MR).

La zéolithe est une zéolithe de type aluminosilicate ayant un rapport global Si/Al supérieur à 10, de préférence supérieur à 20 et de manière préférée, supérieur à 30.

La zéolithe est choisie parmi la ferriérite, le ZSM-5, la ZSM-12, la NU-86, le mordénite, la ZSM-22, la NU-10, la ZBM-30, la ZSM-11, la ZSM-57, l'IZM-2, l'ITQ-6 et l'IM-5, prises seules ou en mélange. De manière préférée, la zéolithe est choisi parmi la ferriérite, la ZSM-5 et la ZSM-12, prises seules ou en mélange. De manière très préférée, la zéolithe est la ZSM-5.

Selon l'invention, ladite zéolithe est mise en forme avec un liant aluminique. Ledit liant aluminique est choisi parmi les hydrates d'alumine et de manière préférée parmi l'hydrargillite, la gibbsite, la nordstrandite, la bayerite, la boehmite ou pseudo-boehmite et les gels d'alumine amorphe, pris seuls ou en mélange, ou parmi les alumines de transition choisies parmi les alumines chi, kappa, rho, eta, theta, gamma et delta, seules ou en mélange. De manière très préférée, ledit liant aluminique est la boehmite.

Ledit catalyseur est constitué d'une zéolithe possédant des ouvertures de pores contenant 10 ou 12 atomes d'oxygènes (10MR ou 12MR) mise en forme avec un liant aluminique. De préférence, ledit catalyseur ne comprend pas de promoteur métallique.

Ledit catalyseur comprend et est de préférence constitué de 20 à 70% poids, et de manière préférée entre 30 et 65% poids de zéolithe possédant des ouvertures de pores contenant 10 ou 12 atomes d'oxygène (10MR ou 12MR) et de 30 à 80% poids et de manière préférée entre 35 et 70% poids de liant aluminique, les pourcentages poids étant exprimés par rapport à la masse totale dudit catalyseur.

Selon l'invention, ledit catalyseur subit, avant son utilisation dans ledit procédé d'oligomérisation, au moins une étape de traitement thermique opérée sous un flux d'air humide contenant entre 1 et 20% poids d'eau, de préférence entre 2 et 10% et de façon préférée entre 4 et 8%, à un débit d'air humide compris entre 0,1 et 10 NL/h/g_{cat}, de préférence entre 0,5 et 2NL/h/g_{cat}, à une température comprise entre 450 et 700°C, de préférence entre 500 et 650°C, de manière préférée entre 550 et 650°C et pendant une durée comprise entre 1 et 10h, et de préférence entre 2 et 6h, ladite étape de traitement thermique étant opérée après l'étape de mise en forme de la zéolithe et du liant aluminique.

Le débit d'air humide est exprimé en normaux litres d'air par heure et par gramme de catalyseur.

Selon la présente invention, on entend par air humide, un flux d'air comprenant entre 1 et 20% poids d'eau et de préférence entre 2 et 10% et de façon préférée entre 4 et 8% poids d'eau.

Les conditions opératoires spécifiques de ladite étape de traitement thermique permettent d'augmenter le nombre de cycles pendant la durée de vie du catalyseur, et/ou l'obtention d'une productivité accrue pendant la durée de vie du catalyseur.

Ladite étape de traitement thermique est en général précédée de plusieurs étapes d'un procédé de préparation dudit catalyseur.

Le catalyseur utilisé dans le procédé selon l'invention peut avantageusement être préparé selon un procédé de préparation comprenant au moins les étapes suivantes :
a) une étape de mélange d'au moins une poudre de zéolithe avec au moins une poudre d'au moins un liant aluminique et au moins un solvant,
b) une étape de mise en forme du mélange obtenu à l'issue de l'étape a),
c) une étape de séchage du matériau mis en forme obtenu à l'issue de l'étape b),
d) éventuellement une étape de calcination sous air sec du matériau séché obtenu à l'issue de l'étape c),
e) une étape de traitement thermique selon l'invention, dudit matériau séché et éventuellement calciné.

De préférence, ladite étape a) consiste en le mélange d'au moins une poudre d'au moins une zéolithe avec au moins une poudre d'au moins un liant aluminique et au moins un solvant pour obtenir un mélange réactionnel qui doit être mis en forme.
Éventuellement, au moins un adjuvant organique est également mélangé au cours de l'étape a).
De manière préférée, au moins ladite poudre d'au moins un liant aluminique et éventuellement au moins ledit adjuvant organique peuvent être mélangés sous forme de poudre ou en solution dans ledit solvant.
Ledit solvant est avantageusement choisi parmi l'eau, l'éthanol, les alcools et les amines, seuls ou en mélange. De préférence, ledit solvant est l'eau.
Il est tout à fait envisageable de procéder à des mélanges de plusieurs poudres de zéolithe et/ou de poudre de sources d'alumines différentes.

L'ordre dans lequel le mélange des poudres d'au moins une zéolithe, d'au moins un liant aluminique et éventuellement d'au moins un adjuvant organique dans le cas où ceux-ci sont mélangés sous forme de poudres, avec au moins un solvant est réalisé est indifférent.
Le mélange desdites poudres et dudit solvant peut avantageusement être réalisé en une seule fois.
Les ajouts de poudres et de solvant peuvent également avantageusement être alternés.

De préférence, lesdites poudres d'au moins une zéolithe, d'au moins un liant aluminique et éventuellement d'au moins un adjuvant organique, dans le cas ou ceux-ci sont mélangés sous forme de poudres, sont d'abord pré-mélangées, à sec, avant l'introduction du solvant. Lesdites poudres pré-mélangées sont ensuite avantageusement mises en contact avec ledit solvant, au moins ledit adjuvant organique pouvant éventuellement être en solution ou suspension dans ledit solvant.
La mise en contact avec ledit solvant conduit à l'obtention d'un mélange réactionnel qui est ensuite avantageusement malaxé.

Les poudres sont avantageusement malaxées en présence d'un solvant, de préférence l'eau, dans lequel un acide, de préférence choisi parmi l'acide acétique, l'acide chlorhydrique, l'acide sulfurique, l'acide formique, l'acide citrique et l'acide nitrique, seul ou en mélange, peut avantageusement être dissout afin d'obtenir une meilleure dispersion du liant. De même, une base, de préférence choisie parmi la soude, la potasse, l'ammoniaque, une amine et un composé à ammonium quaternaire peut avantageusement être ajoutée, dans une étape ultérieure de neutralisation, à la pâte obtenue. La consistance de la pâte est ajustée par le biais de la quantité de solvant.

De préférence, ladite étape a) de mélange est réalisée par malaxage, en batch ou en continu.
Dans le cas où ladite étape a) est réalisée en batch, ladite étape a) est avantageusement réalisée dans un malaxeur de préférence équipé de bras en Z, ou à cames, ou dans tout autre type de mélangeur tel que par exemple un mélangeur planétaire. Ladite étape a) de mélange permet d'obtenir un mélange homogène des constituants pulvérulents.
De préférence, ladite étape a) est mise en œuvre pendant une durée comprise entre 5 et 60 min, et de préférence entre 10 et 50 min. La vitesse de rotation des bras du malaxeur est avantageusement comprise entre 10 et 75 tours/minute, de façon préférée entre 25 et 50 tours/minute.

De préférence, ladite étape b) consiste en la mise en forme du mélange obtenu à l'issue de l'étape a) de mélange.
De préférence, le mélange obtenu à l'issue de l'étape a) de mélange est avantageusement mise en forme par extrusion, ou par pastillage.
Dans le cas où la mise en forme dudit mélange issu de l'étape a) est réalisée par extrusion, ladite étape b) est avantageusement réalisée dans une extrudeuse piston, mono-vis ou bi-vis.
Dans ce cas, un adjuvant organique peut éventuellement être ajouté dans l'étape a) de mélange. La présence dudit adjuvant organique facilite la mise en forme par extrusion.. La présence dudit adjuvant organique facilite la mise en forme par extrusion..Ledit adjuvant organique est avantageusement choisi parmi les polyéthylène glycols, les acides aliphatiques mono-carboxyliques, les composés aromatiques alkylés, les sels d'acide sulphonique, les acides gras, la polyvinyl pyrrolidone, l'alcool polyvinylique, la méthylcellulose, les dérivés de cellulose, les dérivés de type cellulose hydroxyéthylée, la carboxyméthylcellulose, les polyacrylates, les polymétacrylates, le polyisobutène, le polytétrahydrofurane, l'amidon, les polymères de type polysaccharide (comme la gomme de xanthane), le scléroglucane, les lignosulfonates et les dérivés de galactomannane, pris seul ou en mélange. La proportion dudit adjuvant organique est avantageusement comprise entre 0 et 20% en poids, de préférence entre 0 et 10% en poids et de manière préférée entre 0 et 7% en poids, par rapport à la masse totale dudit matériau.

Dans le cas où ledit procédé de préparation est mis en œuvre en continu, ladite étape a) de mélange peut être couplée avec l'étape b) de mise en forme par extrusion dans un même équipement. Selon cette mise en œuvre, l'extrusion de la "formulation homogène" nommé aussi "pâte malaxée" peut être réalisée soit en extrudant directement en bout de malaxeur continu de type bi-vis par exemple, soit en reliant un ou plusieurs malaxeurs batch à une extrudeuse. La géométrie de la filière, qui confère leur forme aux extrudés, peut être choisie parmi les filières bien connues de l'Homme du métier. Elles peuvent ainsi être par exemple, de forme cylindrique, multilobée (tels que bilobés, trilobés, quadrilobés, cannelés...) ou à fentes, elles peuvent éventuellement être telles que le catalyseur se présente sous la forme de poudres concassées, de tablettes, d'anneaux, de billes, de roues.
Dans le cas où la mise en forme du mélange réactionnel issu de l'étape a) est réalisée par extrusion, la quantité de solvant mise en œuvre dans l'étape a) de mélange est ajustée de façon à obtenir, à l'issue de cette étape et quelle que soit la variante mise en œuvre, une pâte qui ne coule pas mais qui n'est pas non plus trop sèche afin de permettre son extrusion dans des conditions convenables de pression bien connues de l'homme du métier et dépendantes de l'équipement d'extrusion utilisé.

De préférence, ladite étape c) consiste en un séchage dudit matériau mis en forme obtenu à l'issue de l'étape b) à une température comprise entre 50 et 200°C et de préférence entre 80 et 150°C, pendant une durée comprise entre 1 et 24 heures.
De préférence, ladite étape de séchage est effectuée sous air.

Le matériau séché issu de l'étape c) subit ensuite éventuellement une étape de calcination à une température comprise entre 450 et 700°C, de préférence entre 540 et 700°C pendant une durée comprise entre 1 et 6 h et de préférence comprise entre 2 et 4h.
Ladite étape de calcination est mise en œuvre sous un flux gazeux comprenant de l'oxygène.

Le matériau séché et éventuellement calciné subit ensuite ladite étape e) de traitement thermique selon l'invention. De préférence ladite étape e) de traitement thermique est une étape de traitement hydrothermal. Selon l'invention, ladite étape e) est opérée sous un flux d'air humide contenant entre 1 et 20% poids d'eau, de préférence entre 2 et 10% et de façon préférée entre 4 et 8%, à un débit d'air humide compris entre 0,1 et 10 NL/h/g_{cat}, de préférence entre 0,5 et 2NL/h/g_{cat}, à une température comprise entre 450 et 700°C, de préférence entre 500 et 650°C, de manière préférée entre 550 et 650°C et pendant une durée comprise entre 1 et 10h, et de préférence entre 2 et 6h, ladite étape de traitement thermique étant opérée après l'étape de mise en forme de la zéolithe et du liant aluminique.
Les conditions opératoires spécifiques de ladite étape de traitement thermique permettent d'augmenter le nombre de cycles pendant la durée de vie du catalyseur, et/ou permettent l'obtention d'une productivité accrue pendant la durée de vie du catalyseur.

Selon une variante, ladite étape de traitement thermique e) selon l'invention peut avantageusement être réalisée directement après l'étape c) de séchage. Dans ce cas, la calcination a lieu dans l'étape de traitement thermique selon l'invention.
Selon une autre variante, ladite étape de traitement thermique selon l'invention peut avantageusement être réalisée après l'étape d) de calcination.
Dans tous les cas, ladite étape e) de traitement thermique est réalisée après l'étape de mise en forme du mélange obtenu à l'issue de l'étape a).

A l'issue du procédé de préparation du catalyseur utilisé dans le procédé selon l'invention, le matériau obtenu se présente sous forme d'extrudés. Cependant, il n'est pas exclu que lesdits matériaux obtenus soient ensuite, par exemple introduits dans un équipement permettant d'arrondir leur surface, tel qu'un drageoir ou tout autre équipement permettant leur sphéronisation (cette étape pouvant se positionner juste après l'étape b) de mise en forme ou ultérieurement). Ainsi, il est avantageusement utilisé sous la forme d'extrudés cylindriques ou polylobés tels que bilobés, trilobés, polylobés de forme droite ou torsadée, mais peut éventuellement être fabriqué et employé sous la forme de poudres concassées, de tablettes, d'anneaux, de billes, de roues, de sphères. De préférence, ledit catalyseur est sous forme d'extrudés de taille comprise entre 1 et 10 mm.

La charge oléfinique traitée dans le procédé d'oligomérisation selon l'invention comprend des oléfines comprenant au moins 3 atomes de carbone et de préférence les coupes oléfiniques en C3-C10 contenant au moins 30% en poids d'oléfines linéaires ou branchées. De préférence, ladite charge oléfinique traitée dans le procédé d'oligomérisation selon l'invention est issue d'une unité de vapocraquage, d'une unité FCC, d'une unité de Fischer Tropsch, d'une unité de production d'oléfines à partir de méthanol ou d'une unité de déshydratation des alcools. Ladite charge oléfinique traitée dans le procédé d'oligomérisation selon l'invention peut avantageusement subir une étape de prétraitement avant d'être utilisée dans le procédé d'oligomérisation selon l'invention. Ladite étape de prétraitement permet d'éliminer tout composé pouvant occasionner un empoisonnement du catalyseur d'oligomérisation.

Conformément à l'invention, le procédé d'oligomérisation selon l'invention opère avantageusement à une température comprise entre 150 et 350°C, de préférence entre 200 et 320°C, et de manière préférée entre 230 et 310°C, à une pression comprise entre 0,2 et 10 MPa et de préférence entre 0,4 et 7 MPa et à une vitesse pondérale horaire comprise entre 0,1 et 8h-1, de préférence entre 0,1 et 5h-1 et de manière préférée entre 0,5 et 1,5 h-1.

Le procédé d'oligomérisation selon l'invention peut avantageusement être opéré selon divers modes. Dans un mode de réalisation préféré, le catalyseur est avantageusement disposé en lit fixe dans un réacteur vertical, sous une des formes décrites ci-dessus, et la charge est injectée sous forme liquide, les conditions de température et de pression étant choisies de manière à permettre à la réaction de se dérouler en une phase liquide unique.

La réaction d'oligomérisation s'accompagne par la formation d'un dépôt de coke à la surface du catalyseur et d'une désactivation du catalyseur qui peut pendant une période de temps restreinte être compensée par une augmentation de la température de réaction. A la fin de cette phase, appelé cycle du catalyseur, il est nécessaire de régénérer le catalyseur pour restaurer son activité. La régénération peut s'effectuer par calcination sous atmosphère oxydante, telle que par exemple en présence du dioxygène de l'air. La réaction de calcination peut être résumée par l'équation suivante : CₙH₂ₘ + (n+m/2) O₂ → n CO₂ + m H₂O. Cette réaction, fortement exothermique, produit donc des quantités importantes de dioxyde de carbone et d'eau et s'effectue à des niveaux de températures élevés et généralement entre 200 et 600°C. De manière générale, la vitesse de réaction et donc l'exothermie de la réaction sont limitées par diminution de la concentration de l'oxydant dans le gaz de régénération via l'introduction d'un diluant inerte tel que par exemple le diazote (N₂) ou le dioxyde de carbone (CO₂). Ainsi le gaz de combustion en sortie d'unité, contenant un mélange de N₂, CO₂ et H₂O est partiellement recyclé pour jouer ce rôle de diluant. Ces conditions de régénération imposent des contraintes supplémentaires au catalyseur d'oligomérisation notamment en terme de résistance hydrothermale pour pouvoir limiter au maximum les désactivations irréversibles, telles que par exemple les modifications morphologiques du catalyseur, causées par la vapeur d'eau lors de la phase de régénération.

Le procédé d'oligomérisation selon l'invention permet la production d'un effluent qui peut avantageusement être séparé en au moins une coupe légère bouillant à une température inférieure à 150°C et en au moins une coupe bouillant à une température comprise entre 150 et 360°C, appelée coupe distillat, de préférence par distillation. La coupe légère peut avantageusement être recyclée à l'entrée du réacteur d'oligomérisation pour accroître la conversion des oléfines et la proportion de coupe distillat. La coupe bouillant à une température comprise entre 150 et 360°C subit avantageusement une étape d'hydrogénation avant d'être incorporée au pool distillat moyen.

Les exemples ci-après illustrent l'invention sans en limiter la portée.

### Exemples

Tous les exemples de tests catalytiques donnés ci-dessous ont été mis en œuvre dans un réacteur lit fixe. La charge oléfinique a au préalable été prétraitée de façon à éliminer tout composé pouvant occasionner un empoisonnement du catalyseur. A l'issue de l'étape d'oligomérisation de cette charge, le catalyseur est alors régénéré sous atmosphère oxydante afin de commencer un nouveau cycle d'oligomérisation de la charge oléfinique. Les productivités seront déterminées par la quantité de produits formés ayant un point d'ébullition supérieur à 100°C (point d'ébullition supérieur au point d'ébullition maximal de la charge).

L'exploitation des résultats et la comparaison des différents catalyseurs sera réalisée de la façon décrite ci-après. L'objectif intrinsèque d'un catalyseur d'oligomérisation est de convertir un maximum d'oléfines courtes en oléfines supérieures tout au long de sa durée de vie. Pour cela, il doit présenter une productivité initiale suffisamment élevée sur un cycle d'oligomérisation et la maintenir au fur et à mesure des régénérations. Celle-ci peut être optimisée soit par une augmentation de l'activité du catalyseur (augmentation de la productivité horaire) soit par une diminution de sa vitesse de désactivation (augmentation de la durée de cycle). Les paramètres observés seront donc la productivité sur le 1^{er} et 5^{ème} cycle, le nombre de cycles réalisés sur la durée de vie du catalyseur et sa productivité sur cette période.

### Protocole général de test

Pour l'ensemble des catalyseurs préparés selon les exemples décrits ci-dessous, les conditions de test ont été les suivantes :
- Le test d'oligomérisation de la charge oléfinique a été réalisé avec une charge industrielle représentative dont la composition est donnée dans le tableau 1, à une pression P de 5 MPa, à une température de 275°C , à une whsv (vitesse pondérale horaire) de 5h⁻¹ (whsv=débit massique de charge / masse de catalyseur). Le test est arrêté à partir du moment où le catalyseur a perdu la moitié de sa productivité initiale. Le choix d'une température et d'une whsv élevée (5h⁻¹) n'est en soi pas représentatif des conditions opératoires de fonctionnement d'une unité industrielle mais est idéal pour accentuer la vitesse de désactivation du catalyseur et observer plus rapidement les différences de comportement entre catalyseurs.

La productivité d'un cycle sera déterminée par la quantité de produits formés ayant un point d'ébullition supérieur à 100°C (point d'ébullition supérieur au point d'ébullition maximal de la charge).

La charge oléfinique utilisée dans ces exemples est une coupe ex-FCC majoritairement composée d'oléfines et paraffines en C5 dont la composition est donnée dans le tableau 1.

**Tableau 1 : Composition de la charge oléfinique.**

| Type de composé | | % en masse |
|---|---|---|
| Paraffines | C4- | 0.4 |
| | C5 | 27.8 |
| | C6+ | 6.4 |
| Oléfines | C4- | 1.5 |
| | C5 | 52.0 |
| | C6+ | 11.2 |
| Autres (naphtènes, diènes | | 0.7 |

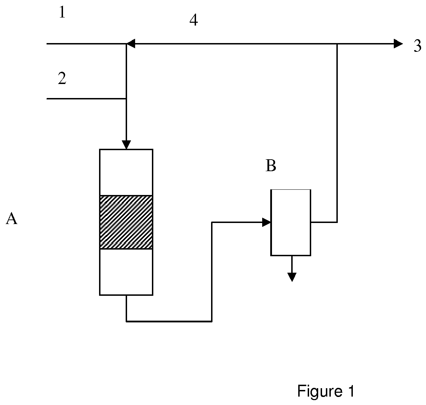

- A la fin de chaque test d'oligomérisation, le catalyseur est régénéré par calcination sous air. Les conditions de régénération ont été choisies de façon à être les plus représentatives d'une régénération industrielle. Les conditions de l'étape de régénération sont décrites en détail dans le tableau 2 et la régénération est réalisée selon le schéma représenté à la figure 1 qui illustre la représentation schématique de la circulation des flux lors des diverses phases.

Après chaque test d'oligomérisation, pour régénérer la catalyseur, on introduit dans le réacteur (A) un débit d'air Q3 représenté sur la figure 1 par le flux 2 en mélange avec une partie d'un gaz de combustion Q1 représenté par le flux 4 qui comprend un mélange de N2, CO2 et H2O et qui est issu de la sortie du réacteur après purge représenté par le flux 3. A la fin de la régénération, un débit de N2 Q2 représenté par le flux 1 est envoyé dans le réacteur en mélange avec le débit Q1 représenté par le flux 4 après purge et passage dans le séparateur (B), pour la phase d'inertage du réacteur.

**Tableau 2 : conditions opératoires de l'étape de régénération des catalyseurs**

| Périodes | Température initiale | Température finale | rampe | Débit de gaz retour Q1 (l/h/gcat) | Débit d'azote Q2 (l/h/gcat) | Débit d'air Q3 (l/h/gcat) | Durée du palier | phénomène |
|---|---|---|---|---|---|---|---|---|
| 1 | 200°C | 350°C | 20°C/h | 1.4 | 0 | 0.6 | 5h | Calcination du coke mou |
| 2 | 350°C | 480°C | 20°C/h | 1.4 | 0 | 0.6 | 5h | |
| 3 | 480°C | 540°C | 20°C/h | 1.4 | 0 | 0.6 | 10h | Calcination du coke dur |
| 4 | 540°C | 590°C | 20°C/h | 1.4 | 0 | 0.6 | 10h | |
| 5 | 590°C | 590°C | --- | 0 | 0 | 2 | 10h | |
| 6 | 590°C | 230°C | 100°C/h | 1.4 | 0.6 | 0 | 2h | Inertage |

Après une première période sous flux d'azote permettant d'évacuer les restes d'hydrocarbures volatils présents dans le réacteur et sur le catalyseur, de l'air est introduit dans l'unité pour initier la régénération du catalyseur. Cet air est dilué grâce à un recyclage partiel du gaz en sortie (taux de recycle=5). Pendant cette phase (périodes 1-4), la température est graduellement augmentée afin de bruler le coke tout en maitrisant l'exotherme de la réaction. Enfin, le coke résiduel est complètement brulé après une dernière étape sous air pur (période 5). L'unité est ensuite inertée sous N₂ pour pouvoir commencer le test d'oligomérisation suivant.
La durée de vie du catalyseur sera estimée d'après le nombre de régénérations successives que le catalyseur aura subies avant que son niveau de productivité sur un cycle n'atteigne un niveau critique fixé arbitrairement à 100g/g_{cat}.
La productivité du catalyseur sur l'ensemble de sa durée de vie sera alors obtenue en faisant la somme des productivités de chaque cycle.

### Exemple 1 : comparatif : cas de référence : catalyseur C1 calciné à 600°C sous air sec

Un catalyseur C1 non-conforme à l'invention en ce qu'il ne subit pas d'étape de traitement hydrothermal selon l'invention est préparé.

Les poudres de boehmite et de zéolithe ZSM-5 (CBV8014 fournie par Zeolyst) sont introduites dans le malaxeur et l'eau acidifiée est ajoutée sous malaxage à 50 tours/min dans un malaxeur batch équipé de bras en Z. Le malaxage acide est poursuivi pendant 30 minutes. Une étape de neutralisation est réalisée par ajout d'une solution ammoniacale et un malaxage pendant 15 minutes. La pâte obtenue est extrudée sur extrudeuse piston à une vitesse de 10mm/min.

Après extrusion, les joncs sont séchés une nuit à 80°C.

Le solide séché obtenu est ensuite calciné à 600°C, pendant 2h avec un débit de 1NL/h/_{gcat} d'air sec, c'est-à-dire sous un flux d'air contenant 0% poids d'eau.

Le catalyseur C1 obtenu est donc comparatif en ce qu'il ne subit pas d'étape de traitement hydrothermal selon l'invention.

Le catalyseur C1 obtenu comprend 60% poids de zéolithe ZSM-5 et 40% poids de liant aluminique, les pourcentages poids étant exprimés par rapport à la masse totale de catalyseur.

### Exemple 2 : préparation du catalyseur C2 selon l'invention

Le catalyseur C2 est préparé de la même manière que le catalyseur C1 jusqu'à l'étape de séchage.

Le solide séché obtenu subit ensuite une étape de traitement hydrothermal selon l'invention. Ladite étape de traitement hydrothermal opère sous un flux d'air humide contenant 2% poids d'eau, à un débit d'air humide de 1 NL/h/g_{cat}, à une température de 600°C et pendant une durée de 2 heures.

Le catalyseur C2 obtenu comprend 60% poids de zéolithe ZSM-5 et 40% poids de liant aluminique, les pourcentages poids étant exprimés par rapport à la masse totale de catalyseur.

### Exemple 3 : préparation du catalyseur C3 selon l'invention

Le catalyseur C3 est préparé de la même manière que le catalyseur C2 à la différence près que le solide séché obtenu subit ensuite une étape de traitement hydrothermal selon l'invention dans les conditions suivantes :
- flux d'air humide contenant 4% poids d'eau,
- débit d'air humide de 1 NL/h/g_{cat},
- température de 600°C et pendant une durée de 2 heures.

Le catalyseur C3 obtenu comprend 60% poids de zéolithe ZSM-5 et 40% poids de liant aluminique, les pourcentages poids étant exprimés par rapport à la masse totale de catalyseur.

### Exemple 4 : préparation du catalyseur C4 selon l'invention

Le catalyseur C4 est préparé de la même manière que le catalyseur C2 à la différence près que le solide séché obtenu subit ensuite une étape de traitement hydrothermal selon l'invention dans les conditions suivantes :
- flux d'air humide contenant 6% poids d'eau,
- débit d'air humide de 1 NL/h/g_{cat},
- température de 600°C et pendant une durée de 2 heures.

Le catalyseur C3 obtenu comprend 60% poids de zéolithe ZSM-5 et 40% poids de liant aluminique, les pourcentages poids étant exprimés par rapport à la masse totale de catalyseur.

### Exemple 5 : comparatif : préparation d'un catalyseur C5 non-conforme.

Un catalyseur C5 non-conforme en ce que la zéolithe non mise en forme avec le liant aluminique est calcinée à 600°C sous air humide, puis mise en forme puis calcinée sous air sec, est préparé.

La zéolithe ZSM-5 (CBV8014 fournie par Zeolyst) subit, avant sa mise en forme avec le liant aluminique, une étape de traitement hydrothermal selon l'invention, dans les conditions suivantes :
- flux d'air humide contenant 4% poids d'eau,
- débit d'air humide de 1 NL/h/g_{cat},
- température de 600°C et pendant une durée de 2 heures.

La zéolithe ainsi obtenue est ensuite mélangée avec la poudre de boehmite dans le malaxeur et l'eau acidifiée est ajoutée sous malaxage à 50 tours/min dans un malaxeur batch équipé de bras en Z. Le malaxage acide est poursuivi pendant 30 minutes. Une étape de neutralisation est réalisée par ajout d'une solution ammoniacale et un malaxage pendant 15 minutes. La pâte obtenue est extrudée sur extrudeuse piston à une vitesse de 10mm/min.

Après extrusion, les joncs sont séchés une nuit à 80°C.

Le solide séché obtenu est ensuite calciné à 600°C pendant 2h sous air sec, c'est-à-dire sous un flux d'air contenant 0% poids d'eau.

Le catalyseur C5 obtenu est donc comparatif en ce que c'est la zéolithe, avant son étape de mise en forme avec le liant aluminique, qui subit l'étape de traitement hydrothermal selon l'invention, et non pas la zéolithe mise en forme, et la zéolithe mise en forme avec le liant aluminique est ensuite calcinée sous air sec, selon un traitement thermique non-conforme à la présente invention.

Le catalyseur C5 obtenu comprend 60% poids de zéolithe ZSM-5 et 40% poids de liant aluminique, les pourcentages poids étant exprimés par rapport à la masse totale de catalyseur.

### Exemple 6 : préparation du catalyseur C6 selon l'invention

Le catalyseur C6 est préparé de la même manière que le catalyseur C2 à la différence près que le solide séché obtenu subit ensuite une étape de traitement hydrothermal selon l'invention dans les conditions suivantes :
- flux d'air humide contenant 10% poids d'eau,
- débit d'air humide de 1 NL/h/g_{cat},
- température de 600°C et pendant une durée de 2 heures.

Le catalyseur C3 obtenu comprend 60% poids de zéolithe ZSM-5 et 40% poids de liant aluminique, les pourcentages poids étant exprimés par rapport à la masse totale de catalyseur.

### Exemple 7 : préparation du catalyseur C7 selon l'invention

Le catalyseur C7 est préparé de la même manière que le catalyseur C2 à la différence près que le solide séché obtenu subit ensuite une étape de traitement hydrothermal selon l'invention dans les conditions suivantes :
- flux d'air humide contenant 20% poids d'eau,
- débit d'air humide de 1 NL/h/g_{cat},
- température de 600°C et pendant une durée de 2 heures.

Le catalyseur C3 obtenu comprend 60% poids de zéolithe ZSM-5 et 40% poids de liant aluminique, les pourcentages poids étant exprimés par rapport à la masse totale de catalyseur.

### Exemple 8 : préparation du catalyseur C8 non conforme

Le catalyseur C8 est préparé de la même manière que le catalyseur C2 à la différence près que le solide séché obtenu subit ensuite une étape de traitement hydrothermal non conforme à l'invention en ce que l'étape de traitement hydrothermal a lieu sous flux d'air humide contenant 40% poids d'eau. Les conditions sont les suivantes :
- flux d'air humide contenant 40% poids d'eau,
- débit d'air humide de 1 NL/h/g_{cat},
- température de 600°C et pendant une durée de 2 heures.

Le catalyseur C3 obtenu comprend 60% poids de zéolithe ZSM-5 et 40% poids de liant aluminique, les pourcentages poids étant exprimés par rapport à la masse totale de catalyseur.

### Exemple 9 : préparation du catalyseur C9 non conforme

Un catalyseur C9 non-conforme en ce qu'il bne subit pas d'étape de traitement hydrothermal selon l'invention est préparé.

Le catalyseur C9 est préparé de la même manière que le catalyseur C1 à la différence près que le solide séché obtenu est ensuite calciné à 550°C pendant 4 heures sous air sec, c'est-à-dire sous un flux d'air contenant 0% poids d'eau, avec un débit d'air sec de 1NL/h/_{gcat}.

Le catalyseur C9 obtenu est donc comparatif en ce qu'il ne subit pas d'étape de traitement hydrothermal selon l'invention.

Le catalyseur C9 obtenu comprend 60% poids de zéolithe ZSM-5 et 40% poids de liant aluminique, les pourcentages poids étant exprimés par rapport à la masse totale de catalyseur.

### Exemple 10 : préparation du catalyseur C10 selon l'invention

Le catalyseur C10 est préparé de la même manière que le catalyseur C2 à la différence près que le solide séché obtenu subit ensuite une étape de traitement hydrothermal selon l'invention dans les conditions suivantes :
- flux d'air humide contenant 20% poids d'eau,
- débit d'air humide de 1 NL/h/g_{cat},
- température de 550°C et pendant une durée de 2 heures.

Le catalyseur C3 obtenu comprend 60% poids de zéolithe ZSM-5 et 40% poids de liant aluminique, les pourcentages poids étant exprimés par rapport à la masse totale de catalyseur.

Les catalyseurs ainsi obtenus des exemples 1 à 10 sont ensuite testés dans le procédé d'oligomérisation décrit ci-dessus et les résultats sont récapitulés dans le tableau 3 ci-dessous.

**Tableau 3 : Résultats.**

| Exemple | Dénomination et conditions de traitement thermique | Productivité lors du 1^{er} cycle (g/g_{cat}) | Productivité après 5 cycles (g/g_{cat}) | Nombre de cycles avant d'atteindre la productivité minimale 100 g/g_{cat} | Productivité sur la durée du test (kg/g_{cat)} |
|---|---|---|---|---|---|
| 1 - Comparatif | C1-600-0% | 202 | 112 | 6 | 1.01 |
| 2 | C2-600-2% | 220 | 120 | 10 | 1.58 |
| 3 | C3-600-4% | 215 | 136 | 11 | 1.73 |
| 4 | C4-600-6% | 210 | 143 | 12 | 1.78 |
| 5- Comparatif | C5-600-0% | 192 | 112 | 6 | 1.00 |
| 6 | C6-600-10% | 170 | 122 | 8 | 1.16 |
| 7 | C7-600-20% | 155 | 125 | 7 | 1.07 |
| 8 - Comparatif | C8-600-40% | 145 | --- | 4 | 0.59 |
| 9 - Comparatif | C9-550-0% | 200 | 110 | 6 | 0.98 |
| 10 | C10-550-20% | 218 | 125 | 8 | 1.32 |

Les résultats obtenus montrent que les catalyseurs ayant subi le traitement hydrothermal selon l'invention, à savoir les catalyseurs C2, C3, C4, C6, C7 et C10 permettent l'obtention d'une productivité sur la durée du test meilleure que celle obtenue pour les catalyseurs C1 et C9 qui n'ont pas subi le traitement hydrothermal selon l'invention.

En particulier, les catalyseurs C2, C3 et C4 selon l'invention permettent à la fois, de gagner en nombre de cycles pendant la durée de vie du catalyseur, et d'obtenir une productivité accrue pendant la durée de vie du catalyseur, par rapport aux catalyseur C1 de référence.

Le catalyseur C8, qui a subi un traitement thermique non conforme à l'invention, sous un flux d'air humide contenant 40% poids d'eau, présente une productivité en forte diminution probablement causée par une forte désalumination de la zéolithe. Le nombre de cycle avant d'atteindre la productivité minimale est donc faible.

Le catalyseur C5 qui a subi un traitement thermique non conforme à l'invention mais dont la zéolithe non encore mise en forme a subi le traitement hydrothermal selon l'invention ne permet pas de gagner en nombre de cycles par rapport au catalyseur de référence C1 qui a subi la même calcination sous air sec mais dont la zéolithe n'a subi aucun traitement hydrothermal. En revanche, le catalyseur C3, dont la zéolithe a subi le même traitement hydrothermal (flux d'air chargé à 4% d'eau) après mise en forme avec le liant présente une meilleure stabilité au cours de sa durée de vie.

## Revendications

1. Procédé d'oligomérisation d'une charge oléfinique comprenant des oléfines comprenant au moins 3 atomes de carbone mettant en oeuvre un catalyseur comprenant au moins une zéolithe possédant des ouvertures de pores contenant 10 ou 12 atomes d'oxygènes (10MR ou 12MR) de type aluminosilicate ayant un rapport global Si/Al supérieur à 10 choisie parmi la ferriérite, le ZSM-5, la ZSM-12, la NU-86, la mordénite, la ZSM-22, la NU-10, la ZBM-30, la ZSM-11, la ZSM-57, l'IZM-2, l'ITQ-6 et l'IM-5, prises seules ou en mélange, mise en forme avec un liant aluminique choisi parmi les hydrates d'alumine choisis parmi l'hydrargillite, la gibbsite, la nordstrandite, la bayerite, la boehmite ou pseudo-boehmite et les gels d'alumine amorphe, pris seuls ou en mélange et les alumines de transition choisies parmi les alumines chi, kappa, rho, eta, theta, gamma et delta, seules ou en mélange, ledit catalyseur comprenant de 20 à 70% poids, de zéolithe possédant des ouvertures de pores contenant 10 ou 12 atomes d'oxygènes (10MR ou 12MR) et de 30 à 80% poids de liant aluminique, les pourcentages poids étant exprimés par rapport à la masse totale dudit catalyseur, ledit catalyseur ayant subi, avant son utilisation dans ledit procédé d'oligomérisation, au moins une étape de traitement thermique opérée sous un flux d'air humide contenant entre 1 et 20% poids d'eau, sous un débit d'air humide compris entre 0,1 et 10 NL/h/g_{cat}, à une température comprise entre 450 et 700°C et pendant une durée comprise entre 1 et 10h, ladite étape de traitement thermique étant opéré après l'étape de mise en forme de la zéolithe avec le liant aluminique, ledit procédé d'oligomérisation opérant à une température comprise entre 150 et 350°C, à une pression comprise entre 0,2 et 10 MPa et à une vitesse pondérale horaire comprise entre 0,1 et 8h-1.

2. Procédé selon la revendication 1 dans lequel la charge oléfinique comprend les coupes oléfiniques en C3-C10 contenant au moins 30% en poids d'oléfines linéaires ou branchées.

3. Procédé selon l'une des revendications 1 ou 2 dans lequel la zéolithe est choisi parmi la ferriérite, la ZSM-5 et la ZSM-12, prises seules ou en mélange.

4. Procédé selon l'une des revendications 1 à 3 dans lequel la zéolithe est la ZSM-5.

5. Procédé selon l'une des revendications 1 à 4 dans lequel ledit liant aluminique est la boehmite.

6. Procédé selon l'une des revendications 1 à 5 dans lequel ladite étape de traitement thermique opère sous un flux d'air humide contenant entre 2 et 10% poids d'eau.

7. Procédé selon la revendication 6 dans lequel ladite étape de traitement thermique opère sous un flux d'air humide contenant entre 4 et 8% poids d'eau.

8. Procédé selon l'une des revendications 1 à 7 dans lequel ladite étape de traitement thermique opère à un débit d'air humide compris entre 0,5 et 2NL/h/g_{cat}.

9. Procédé selon l'une des revendications 1 à 8 dans lequel ladite étape de traitement thermique opère à une température comprise entre 500 et 650°C.

10. Procédé selon l'une des revendications 1 à 9 dans lequel ladite étape de traitement thermique opère pendant une durée comprise entre 2 et 6h.

## Patentansprüche

1. Verfahren zur Oligomerisation einer Olefin-Charge, umfassend Olefine, umfassend mindestens 3 Kohlenstoffatome, das einen Katalysator einsetzt, umfassend mindestens einen Zeolithen, der Porenöffnungen umfasst, die 10 oder 12 Sauerstoffatome (10MR oder 12MR) vom Aluminosilikat-Typ enthalten, mit einem globalen Verhältnis Si/Al größer als 10, ausgewählt unter Ferrierit, ZSM-5, ZSM-12, NU-86, Mordenit, ZSM-22, NU-10, ZBM-30, ZSM-11, ZSM-57, IZM-2, ITQ-6 und IM-5 allein oder im Gemisch, geformt mit einem Aluminiumbindemittel, ausgewählt unter den Aluminiumoxid-Hydraten, ausgewählt unter Hydrargillit, Gibbsit, Nordstrandit, Bayerit, Boehmit oder Pseudo-Boehmit und den Gelen von amorphem Aluminiumoxid allein oder im Gemisch und den Übergangs-Aluminiumoxiden, ausgewählt unter den Chi-, Kappa-, Rho-, Eta-, Theta-, Gamma- und Delta-Aluminiumoxiden allein oder im Gemisch, wobei der Katalysator 20 bis 70 Gew.-% Zeolith, das Porenöffnungen besitzt, die 10 oder 12 Sauerstoffatome (10MR oder 12MR) enthalten, und 30 bis 80 Gew.% eines Aluminiumbindemittels besitzt, wobei die Gewichtsprozentsätze bezogen auf die Gesamtmasse des Katalysators ausgedrückt sind, wobei der Katalysator vor seiner Verwendung bei dem Oligomerisations-Verfahren mindestens einem Wärmebehandlungsschritt unterzogen wurde, der unter einem feuchten Luftstrom, der zwischen 1 und 20 Gew.-% Wasser enthält, unter einer feuchten Luftmenge zwischen 0,1 und 10 NL/h/g_{cat}, bei einer Temperatur zwischen 450 und 700 °C und während einer Dauer zwischen 1 und 10 h durchgeführt wird, wobei der Wärmebehandlungsschritt nach dem Schritt des Formens des Zeolithen mit dem Aluminiumbindemittel durchgeführt wird, wobei das Oligomerisations-Verfahren bei einer Temperatur zwischen 150 und 350 °C, bei einem Druck zwischen 0,2 und 10 MPa und bei einer stündlichen Gewichts-Geschwindigkeit zwischen 0,1 und 8 h-1 durchgeführt wird.

2. Verfahren nach Anspruch 1, bei dem die Olefin-Charge die Olefin-Fraktionen bei C3-C10 umfasst, die mindestens 30 Gew.-% von linearen oder verzweigten Olefinen enthält.

3. Verfahren nach einem der Ansprüche 1 oder 2, bei dem der Zeolith unter Ferrierit, ZSM-5 und ZSM-12 allein oder im Gemisch ausgewählt ist.

4. Verfahren nach einem der Ansprüche 1 bis 3, bei dem der Zeolith ZSM-5 ist.

5. Verfahren nach einem der Ansprüche 1 bis 4, bei dem das Aluminiumbindemittel Boehmit ist.

6. Verfahren nach einem der Ansprüche 1 bis 5, bei dem der Wärmebehandlungsschritt unter einem feuchten Luftstrom, der zwischen 2 und 10 Gew.-% Wasser enthält, durchgeführt wird.

7. Verfahren nach Anspruch 6, bei dem der Wärmebehandlungsschritt unter einem feuchten Luftstrom, der zwischen 4 und 8 Gew.-% Wasser enthält, durchgeführt wird.

8. Verfahren nach einem der Ansprüche 1 bis 7, bei dem Wärmebehandlungsschritt bei einer feuchten Luftmenge zwischen 0,5 und 2 NL/h/g_{cat} durchgeführt wird.

9. Verfahren nach einem der Ansprüche 1 bis 8, bei dem der Wärmebehandlungsschritt bei einer Temperatur zwischen 500 und 650 °C durchgeführt wird.

10. Verfahren nach einem der Ansprüche 1 bis 9, bei dem der Wärmebehandlungsschritt während einer Dauer zwischen 2 und 6 h durchgeführt wird.

## Claims

1. A process for the oligomerization of an olefinic feed comprising olefins containing at least 3 carbon atoms.employing a catalyst comprising at least one aluminosilicate type zeolite having pore openings containing 10 or 12 oxygen atoms (10MR or 12MR) with an overall Si/Al ratio of more than 10 selected from ferrierite, ZSM-5, ZSM-12, NU-86, mordenite, ZSM-22, NU-10, ZBM-30, ZSM-11, ZSM-57, IZM-2, ITQ-6 and IM-5, used alone or as a mixture, shaped with an alumina binder selected from hydrates of alumina selected from hydrargillite, gibbsite, nordstrandite, bayerite, boehmite or pseudo-boehmite and amorphous alumina gels, used alone or as a mixture, and transition aluminas selected from chi, kappa, rho, eta, theta, gamma and delta aluminas, alone or as a mixture, said catalyst comprising 20% to 70% by weight of zeolite having pore openings containing 10 or 12 oxygen atoms (10MR or 12MR) and 30% to 80% by weight of alumina binder, the percentages by weight being expressed with respect to the total mass of said catalyst, said catalyst having undergone at least one heat treatment step before being used in said oligomerization process carried out in a stream of moist air containing in the range 1% to 20% by weight of water, at a flow rate of moist air in the range 0.1 to 10 NL/h/g_{cat}, at a temperature in the range 450°C to 700°C and for a period in the range 1 to 10 h, said heat treatment step being carried out after the step for shaping the zeolite with the alumina binder, said oligomerization process being carried out at a temperature in the range 150°C to 350°C, at a pressure in the range 0.2 to 10 MPa and at a weight hourly space velocity in the range 0.1 to 8 h⁻¹.

2. The process according to claim 1, in which the olefinic feed comprises C3 - C10 olefinic cuts containing at least 30% by weight of linear or branched olefins.

3. The process according to claim 1 or 2, in which the zeolite is selected from ferrierite, ZSM-5 and ZSM-12, used alone or as a mixture.

4. The process according to claim 1 to 3, in which the zeolite is ZSM-5.

5. The process according to claim 1 to 4, in which said alumina binder is boehmite.

6. The process according to one of claims 1 to 5, in which said heat treatment step is carried out in a stream of moist air containing in the range 2% to 10% by weight of water.

7. The process according to claim 6, in which said heat treatment step is carried out in a stream of moist air containing in the range 4% to 8% by weight of water.

8. The process according to one of claims 1 to 7, in which said heat treatment step is carried out with a flow rate of moist air in the range 0.5 to 2 NL/h/g_{cat}.

9. The process according to one of claims 1 to 8, in which said heat treatment step is carried out at a temperature in the range 500°C to 650°C.

10. The process according to one of claims 1 to 9, in which said heat treatment step is carried out for a period in the range 2 to 6 h.
